# EUROPEAN PATENT APPLICATION

(11) **EP 4 327 812 A1**
(43) Date of publication of application: **28.02.2024**
(21) Application number: 22791055.1
(22) Date of filing: 20.04.2022
(51) Int. Cl.: A61K 31/505, A61P 37/02

(54) **PHARMACEUTICAL COMPOSITION CONTAINING JAK3/JAK1/TBK1 SELECTIVE INHIBITOR AND MEDICAL USE THEREOF**

(30) Priority: 22.04.2021 CN 202110436177
(71) Applicant: Shenzhen Chipscreen Biosciences Co., Ltd., Guangdong 518057 (CN)
(72) Inventor: HUANG, Shengjian, Shenzhen, Guangdong 518057 (CN); LU, Xianping, Shenzhen, Guangdong 518057 (CN); PAN, Desi, Shenzhen, Guangdong 518057 (CN); ZHAO, Yiru, Shenzhen, Guangdong 518057 (CN); ZHANG, Qian, shenzhen, Guangdong 518057 (CN); ZHONG, Hua, Shenzhen, Guangdong 518057 (CN)
(74) Representative: Schnappauf, Georg
(86) International application number: PCT/CN2022/087872
(87) International publication number: WO 2022/222948

(57) **Abstract**

Disclosed is the new use of a JAK3/JAK1/TBK1 selective inhibitor. Animal experiments have proven that the JAK3/JAK1/TBK1 selective inhibitor has significant treatment and improvement effects on autoimmune skin diseases, especially Alopecia areata and inflammatory skin diseases, such as atopic dermatitis.

## Description

### FIELD

The present disclosure relates to the technical field of medicine, and specifically relates to a pharmaceutical composition comprising a JAK3/JAK1/TBK1 selective inhibitor and medical applications thereof.

### BACKGROUND

Autoimmune skin diseases are a kind of skin disease due to immune imbalance caused by immune system's immune response to self-antigen. There are not only many kinds of autoimmune skin diseases, but many immune diseases also include their skin symptoms. Generally speaking, most of the diagnoses are composed of four diseases: psoriasis, which usually involves very itchy scaly skin; scleroderma, which mainly affects the inner skin tissue, and can lead to thickening of the skin near hands and feet; blister, which is usually characterized by large blisters filled with pus; alopecia areata, which mainly affects the scalp and can lead to hair loss. Because the immune system is very complex, the vast majority of autoimmune skin diseases have unknown etiology, have complicated course of disease and are difficult to treat.

In autoimmune skin diseases, one type of autoimmune skin diseases characterized by skin inflammation are inflammatory skin diseases caused by autoimmune disorder. The clinical manifestations of inflammatory skin diseases caused by autoimmune disorder are varied, which can cause various types of skin damage, such as skin redness, swelling, dryness, itching, skin blistering, fluid exudation, scarring or desquamation, contact dermatitis, atopic dermatitis, seborrheic dermatitis, neurodermatitis, insect bite dermatitis and so on.

Atopic dermatitis (AD), also known as atopic eczema, idiopathic dermatitis and specific dermatitis, was once called "ectopic dermatitis" and "hereditary allergic dermatitis", which is a chronic, recurrent and typical inflammatory skin disease. The etiology is not clear, which may be related to heredity, immunity, environmental factors and abnormal skin barrier function. Common clinical manifestations include dry skin, eczema-like rash and intense itching, and the like, which may lead adverse effects on daily life when the condition is severe. According to whether it is complicated with other allergic diseases, atopic dermatitis can be divided into two types: simple type and mixed type. Simple-type dermatitis only manifests as dermatitis. It can further be divided into endogenous type and exogenous type, patients with exogenous type have increased serum total IgE, increased specific IgE level and increased peripheral blood eosinophils, while the above changes were not obvious or absent in patients with endogenous type. Endogenous atopic dermatitis is easy to be missed. In addition to the manifestations of dermatitis, mixed dermatitis is also complicated with allergic asthma, allergic rhinitis, allergic conjunctivitis, and the like. Atopic dermatitis (AD) is one of the common diseases in dermatology department, usually, which first occurs in infancy, and the onset of the disease before the age of one year accounts for 60% of all patients, mostly after two months of birth, and sometimes it can also occur in adulthood. At present, it has become a major global public health problem. In developed countries, the prevalence rate of this disease in children can be as high as 10% - 20%. In China, the total prevalence rate of school-age adolescents (6-20 years old) was 0.70% in 1998, and the prevalence rate of preschool children (1-7 years old) in 10 cities was 2.78% in 2002. In recent 30 years, the incidence of AD in the world has gradually increased, and the prevalence rate in developing countries has increased significantly, which may be related to urbanization and industrialization. In recent 20 years, the prevalence rate of atopic dermatitis in China has also gradually increased.

Alopecia areata (AA), commonly known as "ghost shaving head", is a sudden onset of localized patchy hair loss with clear boundaries, no scar and no inflammatory reaction. The etiology is not completely clear, at present, which is mostly considered to be related to emotional stress, endocrine, heredity, autoimmune, and the like. One of the basic etiology is immune dysfunction. The main task of the human immune system is to identify and resist the invasion of external microorganisms. However, in some cases, T-lymphocytes in the immune system, which are dysfunctional, cannot distinguish normal cells, and attack hair follicles to produce specific immune responses, and destroy the structure of normal hair follicles, resulting in abnormal hair growth, reduced pigment production, and patchy alopecia. The other one of the basic etiology is heredity. Studies show that 25% of patients with alopecia areata have a family history of this disease. Monozygotic twins with identical genes are far more likely to suffer from alopecia areata at the same time than fraternal twins, which suggests that it is related to heredity. One of inducing factors is mental and psychological factors. Psychic stress caused by sleep disorder, frustration, depression, anxiety, and the like, often induces or aggravates alopecia areata. Another one of inducing factors is basic diseases that affect immunity. If someone suffers from the following diseases, the immune function of the human will also be affected, and the possibility of alopecia areata will be higher than that of the general population. The diseases include atopic dermatitis, autoimmune thyroid disease, chronic lymphocytic thyroiditis (Hashimoto's disease), subacute thyroiditis, vitiligo, lupus erythematosus, sicca syndrome, diabetes, Down's syndrome, and the like. Another one of inducing factors is virus infection, using of certain drugs, vaccination and other factors, which may cause immune dysfunction, thus inducing alopecia areata.

WO2016041472A1 discloses the structural general formula of an aromatic heterocyclic compound as a selective JAK3 and/or JAK1 kinase inhibitor, and discloses the specific structures of a series of specific compounds, including
N-(3-((5-chloro-2-((4-fluoro-3-(N-methylacrylamido)phenyl)amino)-4-pyrimidinyl)amino)propyl )-4-cyanobenzamide;
N-(3-(5-chloro-2-(4-fluoro-3-(N-methylacrylamido)phenylamino)pyrimidinyl-4-amino)propyl)-4 -trifluoromethyl-benzamide;
N-(3-(5-chloro-2-(4-fluoro-3-(N-methylacrylamido)phenylamino)pyrimidinyl-4-amino)propyl)-4 -fluorobenzamide;
N-(3-(5-chloro-2-(4-fluoro-3-(N-methylacrylamido)phenylamino)pyrimidinyl-4-amino)propyl)-4 -dimethylamino benzamide;
N-(3-(2-(3-acrylamido-4-fluoro-phenylamino)-5-chloropyrimidinyl-4-amino)propyl)-4-fluoroben zamide, and further discloses the kinase activity data of the above compounds. At present, there have been no reports of the above compounds being used in the treatment of autoimmune skin diseases, for example alopecia areata and inflammatory skin diseases (such as atopic dermatitis).

### SUMMARY

In view of this, the purpose of the present disclosure is to provide a new use of JAK3/JAK1/TBK1 selective inhibitor.

In order to achieve the purpose of the present disclosure, the present disclosure provides the following technical embodiments:
Use of a JAK3/JAK1/TBK1 selective inhibitor in manufacture of a medicament for treating/preventing/ameliorating an autoimmune skin disease or in treating/preventing/ameliorating an autoimmune skin disease.

Preferably, the JAK3/JAK1/TBK1 selective inhibitor is selected from the following compounds, or a pharmaceutically acceptable salt thereof, or a stereoisomer thereof: N-(3-((5-chloro-2-((4-fluoro-3-(N-methylacrylamido)phenyl)amino)-4-pyrimidinyl)amino)propyl )-4-cyanobenzamide;
N-(3-(5-chloro-2-(4-fluoro-3-(N-methylacrylamido)phenylamino)pyrimidinyl-4-amino)propyl)-4 -trifluoromethyl-benzamide;
N-(3-(5-chloro-2-(4-fluoro-3-(N-methylacrylamido)phenylamino)pyrimidinyl-4-amino)propyl)-4 -fluorobenzamide;
N-(3-(5-chloro-2-(4-fluoro-3-(N-methylacrylamido)phenylamino)pyrimidinyl-4-amino)propyl)-4 -dimethylamino benzamide; and
N-(3-(2-(3-acrylamido-4-fluoro-phenylamino)-5-chloropyrimidinyl-4-amino)propyl)-4-fluoroben zamide.

Preferably, the JAK3/JAK1/TBK1 selective inhibitor is selected from N-(3-((5-chloro-2-((4-fluoro-3-(N-methylacrylamido)phenyl)amino)-4-pyrimidinyl)amino)propyl )-4-cyanobenzamide, or a pharmaceutically acceptable salt thereof, or a stereoisomer thereof.

Preferably, the JAK3/JAK1/TBK1 selective inhibitor is N-(3-((5-chloro-2-((4-fluoro-3-(N-methylacrylamido)phenyl)amino)-4-pyrimidinyl)amino)propyl )-4-cyanobenzamide.

Preferably, the JAK3/JAK1/TBK1 selective inhibitor is in a preparation unit dose of 20-600 mg.

Preferably, the administration dosage of the JAK3/JAK1/TBK1 selective inhibitor is 20-600 mg/time/day.

Correspondingly, the purpose of the present disclosure is also to provide a pharmaceutical composition which can be used for treating/preventing/ameliorating an autoimmune skin disease. In order to achieve the above purpose of the disclosure, the present disclosure provides the following technical embodiments:
A pharmaceutical composition for treating/preventing/ameliorating an autoimmune skin disease, which comprises a therapeutically effective amount of JAK3/JAK1/TBK1 selective inhibitor. Preferably, the JAK3/JAK1/TBK1 selective inhibitor is selected from the following compounds, or a pharmaceutically acceptable salt thereof, or a stereoisomer thereof:
N-(3-((5-chloro-2-((4-fluoro-3-(N-methylacrylamido)phenyl)amino)-4-pyrimidinyl)amino)propyl )-4-cyanobenzamide;
N-(3-(5-chloro-2-(4-fluoro-3-(N-methylacrylamido)phenylamino)pyrimidinyl-4-amino)propyl)-4 -trifluoromethyl-benzamide;
N-(3-(5-chloro-2-(4-fluoro-3-(N-methylacrylamido)phenylamino)pyrimidinyl-4-amino)propyl)-4 -fluorobenzamide;
N-(3-(5-chloro-2-(4-fluoro-3-(N-methylacrylamido)phenylamino)pyrimidinyl-4-amino)propyl)-4 -dimethylamino benzamide; and
N-(3-(2-(3-acrylamido-4-fluoro-phenylamino)-5-chloropyrimidinyl-4-amino)propyl)-4-fluoroben zamide.

Preferably, the JAK3/JAK1/TBK1 selective inhibitor is selected from N-(3-((5-chloro-2-((4-fluoro-3-(N-methylacrylamido)phenyl)amino)-4-pyrimidinyl)amino)propyl )-4-cyanobenzamide, or a pharmaceutically acceptable salt thereof, or a stereoisomer thereof.

Preferably, the JAK3/JAK1/TBK1 selective inhibitor is N-(3-((5-chloro-2-((4-fluoro-3-(N-methylacrylamido)phenyl)amino)-4-pyrimidinyl)amino)propyl )-4-cyanobenzamide.

Preferably, the JAK3/JAK1/TBK1 selective inhibitor is in a preparation unit dose of 20-600 mg.

Preferably, the pharmaceutical composition further comprises a pharmaceutically acceptable adjuvant. Preferably, the pharmaceutically acceptable adjuvant is selected from the group consisting of a lubricant, an adhesive, a filler, a preservative, a surfactant, a colorant, a flavoring agent, an emulsifier, a suspending agent, a diluent, a gelling agent, a disintegrant, a pH modifier, a solubilizer, and a mixture thereof. It is known to those skilled in the art that these adjuvants may be appropriately selected according to suitable dosage forms, and their contents can be changed according to specific needs.

In one aspect, the pharmaceutical composition can be injection, tablet, pill, lozenge, soft capsule, hard capsule, granule, powder, solution, suspension, syrup, sustained-release preparation and any other suitable dosage form.

In one aspect, the medicament of the present disclosure can be administered orally.

In one aspect, the drug of the present disclosure can be administered parenterally, for example, intraperitoneal, intramuscular, intra-arterial, intravenous, subcutaneous, intradermal and other routes.

Another embodiment of the present disclosure is the use of the pharmaceutical composition for treating/preventing/ameliorating an autoimmune skin disease in the manufacture of a medicament for treating/preventing/ameliorating an autoimmune skin disease or in treating/preventing/ameliorating an autoimmune skin disease.

Correspondingly, the purpose of the present disclosure is further to provide a kit containing any of the above-mentioned pharmaceutical composition which can be used for treating/preventing/ameliorating an autoimmune skin disease.

Correspondingly, the purpose of the present disclosure is also to provide a treatment method for treating/preventing/ameliorating an autoimmune skin disease, which comprises administering a therapeutically/prophylactically/amelioratively effective amount of JAK3/JAK1/TBK1 selective inhibitor to a subject in need thereof. Preferably, the JAK3/JAK1/TBK1 selective inhibitor is selected from the following compounds, or a pharmaceutically acceptable salt thereof, or a stereoisomer thereof:
N-(3-((5-chloro-2-((4-fluoro-3-(N-methylacrylamido)phenyl)amino)-4-pyrimidinyl)amino)propyl )-4-cyanobenzamide;
N-(3-(5-chloro-2-(4-fluoro-3-(N-methylacrylamido)phenylamino)pyrimidinyl-4-amino)propyl)-4 -trifluoromethyl-benzamide;
N-(3-(5-chloro-2-(4-fluoro-3-(N-methylacrylamido)phenylamino)pyrimidinyl-4-amino)propyl)-4 -fluorobenzamide;
N-(3-(5-chloro-2-(4-fluoro-3-(N-methylacrylamido)phenylamino)pyrimidinyl-4-amino)propyl)-4 -dimethylamino benzamide;
N-(3-(2-(3-acrylamido-4-fluoro-phenylamino)-5-chloropyrimidinyl-4-amino)propyl)-4-fluoroben zamide.

Preferably, the compound is selected from N-(3-((5-chloro-2-((4-fluoro-3-(N-methylacrylamido)phenyl)amino)-4-pyrimidinyl)amino)propyl )-4-cyanobenzamide, or a pharmaceutically acceptable salt thereof, or a stereoisomer thereof.

Preferably, the compound is N-(3-((5-chloro-2-((4-fluoro-3-(N-methylacrylamido)phenyl) amino)-4-pyrimidinyl)amino)propyl)-4-cyanobenzamide.

Preferably, the administration dosage of the JAK3/JAK1/TBK1 selective inhibitor is 20-600 mg/time/day.

Preferably, the autoimmune skin disease in the present disclosure is inflammatory skin diseases, preferably atopic dermatitis.

Preferably, the autoimmune skin disease in the disclosure is alopecia areata.

### Definition:

The "pharmaceutically acceptable salt" described in the present disclosure refers to an acid addition salt prepared by the reaction of JAK3/JAK1/TBK1 selective inhibitor with a pharmaceutically acceptable acid, or a salt produced by the reaction of a compound with an acidic group and a pharmaceutically acceptable base. The pharmaceutically acceptable acid is preferably selected from inorganic acids (such as hydrochloric acid, sulfuric acid, phosphoric acid or hydrobromic acid, etc.) and organic acids (such as oxalic acid, maleic acid, fumaric acid, malic acid, tartaric acid, citric acid or benzoic acid, etc.); the pharmaceutically acceptable base is preferably selected from sodium hydroxide, potassium hydroxide, calcium hydroxide, sodium carbonate, potassium bicarbonate, ammonia water or ammonium bicarbonate.

In the present disclosure, the stereoisomers include conformational isomers, enantiomers and diastereomers.

In the present disclosure, the "therapeutically/prophylactically/amelioratively effective amount" refers to the dose between minimum effective dose and the extreme dose, which can produce a significant effect on the body without causing a toxic reaction.

**Beneficial effect of the present** disclosure: the present disclosure have proven that the JAK3/JAK1/TBK1 selective inhibitor has significant treatment and improvement effects on autoimmune skin diseases, especially alopecia areata and inflammatory skin disease, such as atopic dermatitis through animal experiments.

### BRIEF DESCRIPTION OF DRAWINGS

Fig. 1 shows the statistical results (spleen/body weight, ear thickness) of general observation and detection analysis at the endpoint of the test in example 1.
Fig. 2 shows the statistical results (IgE, ear epidermal thickness, number of mast cells in the ear epidermis) of serum and histopathology detection and analysis at the endpoint of the test in example 1.
Fig. 3 shows a graph of the rate of change of alopecia areata area in each experimental group during the administration in example 2.
Fig. 4 shows a comparative picture of hair growth and area changes of alopecia areata in alopecia areata areas on the back of mice in each experimental group during the administration of example 2.
Fig. 5 shows the comparative statistical results of spleen weight and spleen/body weight ratio of mice in each experimental group at the endpoint of the test in example 2.

In the figures, "*" represents the comparison between the model group and the blank group, and "#" represents the comparison between the administration group and the model group. "*" and "#" represent p < 0.05, "**" and "# #" represent p < 0.01, and "****" represents p < 0.0001.

### DETAILED DESCRIPTION

The present disclosure discloses the treatment/prevention/amelioration effects of JAK3/JAK1/TBK1 selective inhibitors on autoimmune skin diseases, especially alopecia areata and inflammatory dermatitis, such as atopic dermatitis. Those skilled in the art can make several improvements and modifications to the present disclosure with reference to the contents herein. In particular, it should be pointed out that all similar substitutions and modifications are obvious to those skilled in the art, and they are all considered to be included in the present disclosure. The application and pharmaceutical composition described in the present disclosure have been described by preferred examples, and those skilled in the art can make amendments or suitable changes or combinations on the use and pharmaceutical composition described herein, to realize and apply the technology of the present disclosure without departing from the content, spirit and scope of the present disclosure."

The technical embodiments provided by the present disclosure will be further explained in detail through specific experiments.

Unless otherwise defined, all technical and scientific terms used herein have the same meaning as commonly understood by those of ordinary skill in the art.

### Experimental part:

### experimental materials:

Sample for test: JAK3/JAK1/TBK1 selective inhibitor N-(3-((5-chloro-2-((4-fluoro-3-(N-methylacrylamido)phenyl)amino)-4-pyrimidinyl)amino)propyl)-4-cyanobenzamide, synthesized by Shenzhen Chipscreen Biosciences Co., Ltd.

DNCB (2,4-dinitrochlorobenzene) was purchased from Sigma. Preparation: 1% DNCB: 1g DNCB powder was weighed and added into 100ml acetone-ofive oil solution (4:1), and mixed well; 0.5% DNCB: 0.5g DNCB powder was weighed and added into 100ml acetone-ofive oil solution (4:1), and mixed well.

### Example 1 Treatment and amelioration effects on atopic dermatitis symptoms with JAK3/JAK1/TBK1 selective inhibitors

Drug preparation: appropriate amount of powder of sample for test was weighed accurately and put in a sample preparation tube. Purified water was added and sonication was performed until the sample was completely dissolved, and a concentration of 5 mg/ml (based on active ingredient) was prepared, and it was administered within 15 minutes after preparation.

Experimental animals: Balb/c mice were purchased from Sichuan Academy of Traditional Chinese Medicine, 27 mice in total.

### Experimental method:

Grouping: Balb/c mice (27 mice in total) were randomly divided into three groups, 9 mice in each group, which were blank group, model group and administration group respectively.

Modeling: 300 µl of 1% DNCB was applied to the abdomen of mice in the model group and administration group, and 0.5% DNCB was applied to the ears every other day after 7 d, and lasted for 31 days.

Administration: administration was carried out after 12 days of modeling. Administration group: dosage and frequency of administration: 50 mg/kg (based on active ingredient), twice a day. Model group: the same dose of vehicle was given. The administration cycle was 3 weeks (12 d -31 d). The method of administration: oral gavage.

The modeling and administration information is shown in the figure below.

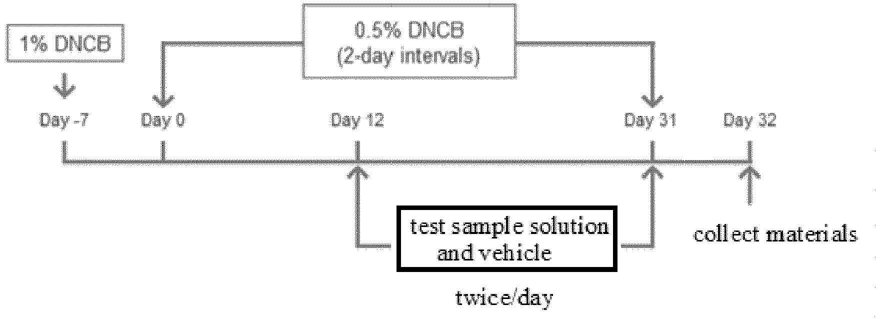

### Detection indicators and detection methods:

(1) General observation: ear thickness and spleen/body weight ratio. At the end point of the test, the body weight of mice was weighed, and the left and right ear thickness(mm) of mice was measured, and then the mice were subjected to gross anatomy, and the spleen of mice was weighed to calculate the spleen/ body weight ratio of mice.
(2) Serum: IgE. At the end point of the test, blood was collected from the orbit of mice, and the total IgE in the serum of mice was detected (ELISA method).
(3) Histopathology: HE staining of the ear (to measure ear epidermal thickness) and toluidine blue staining (to measure the number of mast cells in the ear epidermis).

HE staining of ears: the fixed mouse ear tissue was dehydrated by automatic dehydrator and embedded, and then sliced. After dewaxing the slices to water, hematoxyfin staining was performed until blue appeared; after elution, eosin staining was performed for sealing, and finally microscopy and image acquisition were carried out.

Toluidine blue staining: mouse ear tissue slides were sliced after anti-stripping treatment, and dewaxed it to water routinely; and it was put into 1% toluidine blue aqueous solution that had been preheated to 50 °C and stained for 20 min in an incubator at 56 °C; washed with distilled water; immersed in 70% alcohol for 1 min; differentiated with 95% alcohol; controlled under microscope to render nissl bodies clearly displayed; dehydrated quickly with anhydrous alcohol; transparentized with xylene and sealed with neutral gum. The above specimens were subjected to the procedures of dehydration, trimming, embedding, slicing, staining, sealing, and other procedures, and finally microscopy and image acquisition were carried out.

### Experimental results:

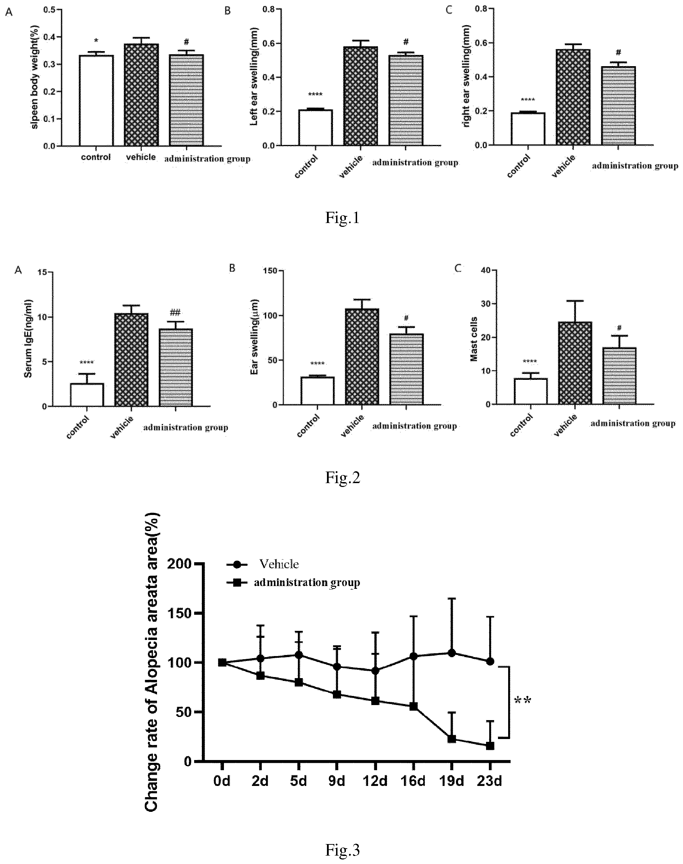

The statistical results of the detection in example 1 are shown in Fig. 1 and Fig. 2. In Fig. 1, A is the statistical result of spleen/body weight ratio (%) at the end point of each experimental group; B is the statistical result of the left ear thickness (left ear swelling, mm) at the end point of each experimental group; C is the statistical result of the right ear thickness (right ear thickness, mm) at the end point of each experimental group. Fig. 1 shows that after DNCB modeling, the spleen/body weight ratio (*) and ear thickness (****) of the vehicle were significantly higher than those of the blank group, suggesting that the modeling was successful. The spleen/body weight ratio (#) and ear thickness (#) (including the left ear and the right ear) of model animals were significantly reduced after administration of the test sample, suggesting that the test sample has the characteristics of significantly relieving atopic dermatitis. In Fig. 2, A is the statistical result of serum IgE (ng/ml) detection and analysis in each experimental group; B is the statistical results of the detection and analysis of ear epidermal swelling (mm) in each experimental group; C is the statistical result of detection and analysis of the number of mast cells in the ear epidermis of each experimental group. Fig. 2 shows that the serum IgE level (****), the ear epidermal thickness (***) and the number of mast cells in ear epidermis (****) were significantly higher than those in the blank group after DNCB modeling, suggesting that the modeling was successful; however, the serum IgE level (##), ear epidermal thickness (#) and the number of mast cells in ear epidermis (#) were significantly decreased in the administration group, suggesting that the test sample has the characteristics of significantly ameliorating atopic dermatitis.

### Example 2 Treatment and amelioration effects on alopecia areata symptoms with JAK3/JAK1/TBK1 selective inhibitors

Drug preparation: appropriate amount of powder of test sample was weighed accurately and put in a sample preparation tube, then add purified water was added and sonification was performed until the sample was completely dissolved. A concentration of 8 mg/ml (based on active ingredient) was prepared, and it was administered within 15 minutes after preparation.

Experimental animals: 16 C57BL/6J mice with alopecia areata, female, 12 weeks old. They were purchased from Beijing Vital River Laboratory Animal Technology Co., Ltd. Animal inclusion criteria: mice with patchy and traceless hair loss, mainly with back hair loss and head hair loss.

### Experimental method:

Grouping and criteria: 14 C57BL/6J mice were enrolled in the groups, and 5 mice in the model group, 9 mice in the administration group (test sample). The overall situation of hair loss in the administration group was more serious than that in the model group.

Administration information: administration group: single dose: 80 mg/kg (based on active ingredient) twice a day for 23d continuously; model group: equal doses of vehicle given. Method of administration: oral gavage.

### Detection indicators and detection methods:

Record of change rate of body weight and alopecia areata area: measured twice a week, weighed and observed with naked eyes on Monday and Thursday.

Spleen size and weight: at the end of the test, the body weight of mice was weighed, and then the mice were subjected to gross anatomy, the spleen of the mice was observed and weighed to calculate the spleen/body weight ratio of mice.

### Experimental results:

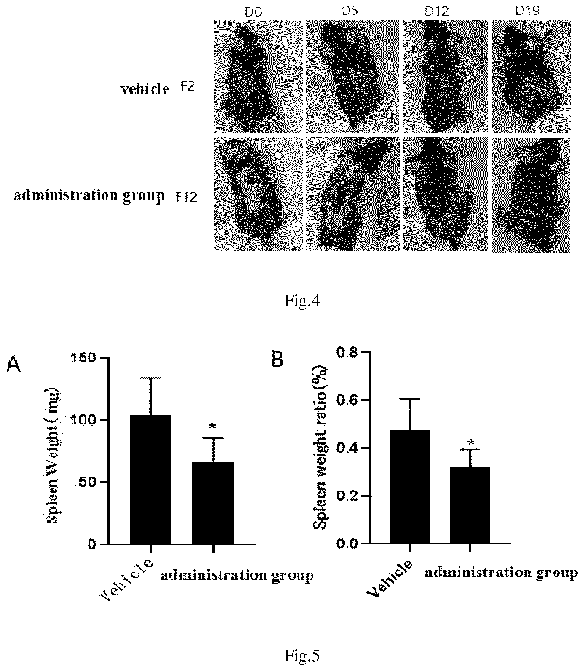

The statistical results of the index detection and analysis of example 2 are shown in Fig. 3, Fig. 4 and Fig. 5. Fig. 3 shows a graph of the change rate of alopecia areata (%) in each experimental group during the administration; Fig. 4 shows a picture of the growth of hair and the change of the alopecia areata area on the back of the mice in each experimental group during the administration (D0 represents the day 0, D5 represents the day 5, and so on). Fig. 3 and Fig. 4 show that the alopecia areata area of animals in the model group remained basically unchanged during the period of administration; the alopecia areata area was significantly ameliorated in the administration group, and the optimal drug efficacy was achieved on the 19^{th}-23^{rd} day; the amelioration of alopecia areata area change in the administration group was significantly better than that in the model group (vehicle) (**, p < 0.01). Fig. 5 shows the statistical results of spleen weighing and spleen/body weight ratio after gross anatomy of mice. Fig. 5 shows the statistical results of spleen weight and spleen/body weight ratio of mice in each experimental group at the end point of the experiment. Fig. 5 shows that the spleen weight and spleen/body weight ratio in the administration group were significantly lower than those in the model group (*, p < 0.05). Combined with the fact that the test sample does not reduce the weight of spleen in normal animals, it suggests that the weight reduction of spleen in this model is related to the mechanism, and the test sample is safe and effective with few toxic and side effects.

The above animal experiments proved that the JAK3/JAK1/TBK1 selective inhibitor provided by the present disclosure has significant therapeutic and ameliorative effects on autoimmune skin diseases, such as atopic dermatitis and alopecia areata.

The present disclosure has been described in detail above, and specifically applying the preferred examples to describe the principles and embodiments of the present disclosure. The description of the above examples are only used to help understand the method and core idea of the present disclosure, including the best mode, and it also makes those skill in the art to be able to practice the present disclosure, including manufacture and use, as well as to implement any of the combined methods. It should be pointed out that for those skilled in the art, without departing from the principle of the present disclosure, several improvements and modifications can be made to the present disclosure, and these improvements and modifications also fall within the scope of protection of the claims of the present disclosure. The scope of the patent protection of the present disclosure is limited by the claims and may include other embodiments that can be conceived by a person skilled in the art. If these other embodiments have structural elements that are not different from the literal expression of the claims, or if they include equivalent structural elements that are not substantially different from the literal expression of the claims, these other embodiments should also be included in the scope of the claims.

## Claims

1. Use of a JAK3/JAK1/TBK1 selective inhibitor in the manufacture of a medicament for treating/preventing/ameliorating an autoimmune skin disease;
preferably, the JAK3/JAK1/TBK1 selective inhibitor is selected from the following compounds, or a pharmaceutically acceptable salt thereof, or a stereoisomer thereof:
N-(3-((5-chloro-2-((4-fluoro-3-(N-methylacrylamido)phenyl)amino)-4-pyrimidinyl)amino)propyl )-4-cyanobenzamide;
N-(3-(5-chloro-2-(4-fluoro-3-(N-methylacrylamido)phenylamino)pyrimidinyl-4-amino)propyl)-4 -trifluoromethyl-benzamide;
N-(3-(5-chloro-2-(4-fluoro-3-(N-methylacrylamido)phenylamino)pyrimidinyl-4-amino)propyl)-4 -fluorobenzamide;
N-(3-(5-chloro-2-(4-fluoro-3-(N-methylacrylamido)phenylamino)pyrimidinyl-4-amino)propyl)-4 -dimethylamino benzamide; and
N-(3-(2-(3-acrylamido-4-fluoro-phenylamino)-5-chloropyrimidinyl-4-amino)propyl)-4-fluoroben zamide;
preferably, the JAK3/JAK1/TBK1 selective inhibitor is N-(3-((5-chloro-2-((4-fluoro-3-(N-methylacrylamido)phenyl)amino)-4-pyrimidinyl)amino)propyl )-4-cyanobenzamide, or a pharmaceutically acceptable salt thereof, or a stereoisomer thereof;
preferably, the JAK3/JAK1/TBK1 selective inhibitor is N-(3-((5-chloro-2-((4-fluoro-3-(N-methylacrylamido)phenyl)amino)-4-pyrimidinyl)amino)propyl )-4-cyanobenzamide;
preferably, the JAK3/JAK1/TBK1 selective inhibitor is in a preparation unit dose of 20-600 mg;
preferably, the autoimmune skin disease is inflammatory skin disease, preferably is atopic dermatitis;
preferably, the autoimmune skin disease is alopecia areata.

2. Use of a JAK3/JAK1/TBK1 selective inhibitor for treating/preventing/ameliorating an autoimmune skin disease;
preferably, the JAK3/JAK1/TBK1 selective inhibitor is selected from the following compounds, or a pharmaceutically acceptable salt thereof, or a stereoisomer thereof:
N-(3-((5-chloro-2-((4-fluoro-3-(N-methylacrylamido)phenyl)amino)-4-pyrimidinyl)amino)propyl )-4-cyanobenzamide;
N-(3-(5-chloro-2-(4-fluoro-3-(N-methylacrylamido)phenylamino)pyrimidinyl-4-amino)propyl)-4 -trifluoromethyl-benzamide;
N-(3-(5-chloro-2-(4-fluoro-3-(N-methylacrylamido)phenylamino)pyrimidinyl-4-amino)propyl)-4 -fluorobenzamide;
N-(3-(5-chloro-2-(4-fluoro-3-(N-methylacrylamido)phenylamino)pyrimidinyl-4-amino)propyl)-4 -dimethylamino benzamide;
N-(3-(2-(3-acrylamido-4-fluoro-phenylamino)-5-chloropyrimidinyl-4-amino)propyl)-4-fluoroben zamide;
preferably, the JAK3/JAK1/TBK1 selective inhibitor is selected from N-(3-((5-chloro-2-((4-fluoro-3-(N-methylacrylamido)phenyl)amino)-4-pyrimidinyl)amino)propyl )-4-cyanobenzamide, or a pharmaceutically acceptable salt thereof, or a stereoisomer thereof;
preferably, the JAK3/JAK1/TBK1 selective inhibitor is N-(3-((5-chloro-2-((4-fluoro-3-(N-methylacrylamido)phenyl)amino)-4-pyrimidinyl)amino)propyl )-4-cyanobenzamide;
preferably, the JAK3/JAK1/TBK1 selective inhibitor is administered in a dosage of 20-600 mg/time/day;
preferably, the autoimmune skin disease is inflammatory skin disease, preferably is atopic dermatitis;
preferably, the autoimmune skin disease is alopecia areata.

3. A pharmaceutical composition for treating/preventing/ameliorating an autoimmune skin disease, wherein the pharmaceutical composition comprises a therapeutically effective amount of JAK3/JAK1/TBK1 selective inhibitor;
preferably, the JAK3/JAK1/TBK1 selective inhibitor is selected from the following compounds, or a pharmaceutically acceptable salt thereof, or a stereoisomer thereof:
N-(3-((5-chloro-2-((4-fluoro-3-(N-methylacrylamido)phenyl)amino)-4-pyrimidinyl)amino)propyl )-4-cyanobenzamide;
N-(3-(5-chloro-2-(4-fluoro-3-(N-methylacrylamido)phenylamino)pyrimidinyl-4-amino)propyl)-4 -trifluoromethyl-benzamide;
N-(3-(5-chloro-2-(4-fluoro-3-(N-methylacrylamido)phenylamino)pyrimidinyl-4-amino)propyl)-4 -fluorobenzamide;
N-(3-(5-chloro-2-(4-fluoro-3-(N-methylacrylamido)phenylamino)pyrimidinyl-4-amino)propyl)-4 -dimethylamino benzamide; and
N-(3-(2-(3-acrylamido-4-fluoro-phenylamino)-5-chloropyrimidinyl-4-amino)propyl)-4-fluoroben zamide;
preferably, the JAK3/JAK1/TBK1 selective inhibitor is N-(3-((5-chloro-2-((4-fluoro-3-(N-methylacrylamido)phenyl)amino)-4-pyrimidinyl)amino)propyl )-4-cyanobenzamide; or a pharmaceutically acceptable salt thereof, or a stereoisomer thereof;
preferably, the JAK3/JAK1/TBK1 selective inhibitor is N-(3-((5-chloro-2-((4-fluoro-3-(N-methylacrylamido)phenyl)amino)-4-pyrimidinyl)amino)propyl )-4-cyanobenzamide;
preferably, the JAK3/JAK1/TBK1 selective inhibitor is in a preparation unit dose of 20-600 mg;
preferably, the autoimmune skin disease is inflammatory skin disease, preferably is atopic dermatitis;
preferably, the autoimmune skin disease is alopecia areata.

4. The pharmaceutical composition according to claim 3, wherein the pharmaceutical composition further comprises a pharmaceutically acceptable adjuvant, preferably, the pharmaceutically acceptable adjuvant is selected from the group consisting of a lubricant, an adhesive, a filler, a preservative, a surfactant, a colorant, a flavoring agent, an emulsifier, a suspending agent, a diluent, a gelling agent, a disintegrant, a pH modifier, a solubilizer, and a mixture thereof.

5. The pharmaceutical composition according to claim 4, wherein the pharmaceutical composition can be tablet, pill, soft capsule, hard capsule, granule, powder, solution, syrup, suspension, injection, lozenge and sustained-release preparation.

6. Use of the pharmaceutical composition for treating/preventing/ameliorating an autoimmune skin disease according to any one of claims 3 to 5 in the manufacture of a medicament for treating/preventing/ameliorating an autoimmune skin disease or in treating/preventing/ameliorating an autoimmune skin disease; preferably, the autoimmune skin disease is inflammatory skin disease, preferably is atopic dermatitis;
preferably, the autoimmune skin disease is alopecia areata.

7. A kit, wherein comprising the pharmaceutical composition for treating/preventing/ameliorating autoimmune skin disease of any one of claims 3-5; preferably, the autoimmune skin disease is inflammatory skin disease, preferably is atopic dermatitis; preferably, the autoimmune skin disease is alopecia areata.

8. A method for treating/preventing/ameliorating an autoimmune skin disease, comprising administering a therapeutically effective amount of JAK3/JAK1/TBK1 selective inhibitor to a subject in need thereof, preferably, the JAK3/JAK1/TBK1 selective inhibitor is selected from the following compounds, or a pharmaceutically acceptable salt thereof, or a stereoisomer thereof:
N-(3-((5-chloro-2-((4-fluoro-3-(N-methylacrylamido)phenyl)amino)-4-pyrimidinyl)amino)propyl )-4-cyanobenzamide;
N-(3-(5-chloro-2-(4-fluoro-3-(N-methylacrylamido)phenylamino)pyrimidinyl-4-amino)propyl)-4 -trifluoromethyl-benzamide;
N-(3-(5-chloro-2-(4-fluoro-3-(N-methylacrylamido)phenylamino)pyrimidinyl-4-amino)propyl)-4 -fluorobenzamide;
N-(3-(5-chloro-2-(4-fluoro-3-(N-methylacrylamido)phenylamino)pyrimidinyl-4-amino)propyl)-4 -dimethylamino benzamide; and
N-(3-(2-(3-acrylamido-4-fluoro-phenylamino)-5-chloropyrimidinyl-4-amino)propyl)-4-fluoroben zamide;
preferably, the compound is N-(3-((5-chloro-2-((4-fluoro-3-(N-methylacrylamido)phenyl) amino)-4-pyrimidinyl)amino)propyl)-4-cyanobenzamide, or a pharmaceutically acceptable salt thereof, or a stereoisomer thereof;
preferably, the JAK3/JAK1/TBK1 selective inhibitor is N-(3-((5-chloro-2-((4-fluoro-3-(N-methylacrylamido)phenyl)amino)-4-pyrimidinyl)amino)propyl )-4-cyanobenzamide;
preferably, the JAK3/JAK1/TBK1 selective inhibitor is administered in a dosage of 20-600 mg/time/day;
preferably, the autoimmune skin disease is inflammatory skin disease, preferably is atopic dermatitis;
preferably, the autoimmune skin disease is alopecia areata.
